# EUROPEAN PATENT APPLICATION

(11) **EP 2 450 111 A1**
(43) Date of publication of application: **09.05.2012**
(21) Application number: 11187155.4
(22) Date of filing: 28.10.2011
(51) Int. Cl.: B06B 1/06

(54) **Ultrasound probe including ceramic layer formed with ceramic elements having different thickness and ultrasound system using the same**

(30) Priority: 04.11.2010 KR 20100108928; 25.10.2011 KR 20110109159
(71) Applicant: Samsung Medison Co., Ltd., Nam-myun, Hongchun-gun Kangwon do 250-870 (KR)
(72) Inventor: Kim, Jung Bae, 135-851 Seoul (KR)
(74) Representative: Schmid, Wolfgang

(57) **Abstract**

An ultrasound probe including a ceramic layer formed with a plurality of ceramic elements having different thicknesses and an ultrasound system using the same are disclosed. The ultrasound probe for transmitting and receiving ultrasound signals includes at least one transducer element having a ceramic layer, wherein the ceramic layer includes a plurality of ceramic elements having different thicknesses.

## Description

### CROSS-REFERENCE TO RELATED APPLICATIONS

The present application claims priority from Korean Patent Application Nos. 10-2010-0108928 and 10-2011-0109159 respectively filed on November 4, 2010 and October 25, 2011.

### TECHNICAL FIELD

The present disclosure generally relates to ultrasound systems, and more particularly to an ultrasound probe including a ceramic layer formed with a plurality of ceramic elements having different thicknesses in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modern high-performance ultrasound systems and techniques are commonly used to produce two or three-dimensional images of internal features of an object (e.g., human organs).

The ultrasound system includes an ultrasound probe configured to transmit an ultrasound signal to a target object and receive an ultrasound signal (i.e., echo signal).

The ultrasound probe includes at least one transducer element for reciprocal conversion between an electric signal and an ultrasound signal.

The transducer element includes a ceramic layer such as a piezoelectric layer for generating an ultrasound signal in response to an electric signal and generating an electric signal in response to an ultrasound signal. The ultrasound signal, which is generated from the ceramic layer, has a low frequency characteristic or a high frequency characteristic according to a characteristic of the ceramic layer. When the ultrasound signal has a high frequency characteristic, a focusing characteristic of the ultrasound signal is fair at a shallow region of the target object so that a high resolution of the ultrasound image may be obtained for the shallow region. However, propagation of the ultrasound signal is difficult at a relatively deep region in the target object. As such, a transmission focusing characteristic of the ultrasound signal may be degraded. Thus, resolution of the ultrasound image at the deep region may be lowered.

However, when the ultrasound signal has a low frequency characteristic, resolution of the ultrasound image at a near region from the ultrasound probe, i.e., a shallow region of the target object, may be lowered compared to the high frequency characteristic. Propagation of the ultrasound signal is easy at a relatively deep region in the target object, and thus, an enhanced ultrasound image may be obtained. Accordingly, an ultrasound probe is required to obtain an enhanced ultrasound image both at shallow and deep regions.

### SUMMARY

An ultrasound probe including a ceramic layer formed with a plurality of ceramic elements having different thicknesses and an ultrasound system using the same are disclosed herein. In one embodiment, by way of non-limiting example, an ultrasound probe for transmitting and receiving ultrasound signals, comprises: at least one transducer element having a ceramic layer, wherein the ceramic layer includes a plurality of ceramic elements having different thicknesses.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG 1 is a block diagram showing an illustrative embodiment of an ultrasound system.
FIG 2 is a cross-sectional view of the ultrasound probe 100 according to one embodiment of the present invention.
FIG 3 is a schematic diagram showing an illustrative embodiment of a ceramic layer in each transducer element.
FIG 4 is a schematic diagram showing profiles of ultrasound transmit beams when a plurality of ceramic elements is formed to gradually have a narrower thickness in both edge directions with respect to a center ceramic element of a ceramic layer.
FIG 5 is a schematic diagram showing profiles of ultrasound transmit beams when a plurality of ceramic elements is formed to gradually have a thicker thickness in both edge directions with respect to a center ceramic element of a ceramic layer.

### DETAILED DESCRIPTION

Hereinafter, embodiments of the present invention will be described by referring to accompanying drawings.

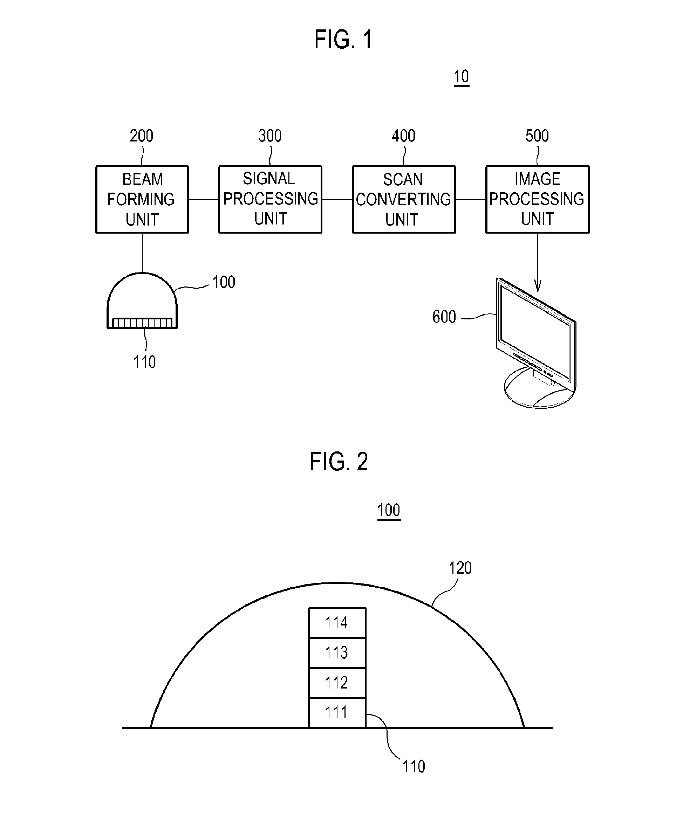

FIG 1 is a block diagram showing an illustrative embodiment of an ultrasound system 10. The ultrasound system 10 includes an ultrasound probe 100, a beam forming unit 200, a signal processing unit 300, a scan converting unit 400, an image processing unit 500 and a display unit 600. The ultrasound system 100 may further include a storage unit (not shown) such as a memory, etc.

The ultrasound probe 100, which includes at least one transducer element 110, is configured to transmit an ultrasound signal in response to a transmit signal that is an electric signal outputted from a transmit signal generating unit (not shown). The ultrasound probe 100 receives an echo signal reflected from the target object. The ultrasound probe 100 is further configured to output a receive signal, which is an electric signal, in response to the receive echo signal.

The beam forming unit 200 is configured to receive-focus the receive signal outputted from the ultrasound probe 110 to form a receive-focused beam. The signal processing unit 300 is configured to perform signal processing such as envelop detection and the like upon the receive-focused beam outputted from the beam forming unit 200 to form ultrasound image data.

The scan converting unit 400 is configured to convert a data format of the ultrasound image data outputted from the signal processing unit 300 to be displayed. The image processing unit 500 is configured to process the image data outputted from the scan converting unit 400 for display on the display unit 600.

FIG 2 is a cross-sectional view of the ultrasound probe 100 according to one embodiment of the present invention. Referring to FIG 2, the ultrasound probe 100 includes at least one transducer element 110. Also, the ultrasound probe 100 further includes lens and membrane cover 120 for focus of the ultrasound signal.

In one embodiment, the transducer element 110 includes a ceramic layer 112 for reciprocal conversion between an electric signal and an ultrasound signal. The ceramic layer 112 may be formed from composite materials of fine crystals, which are arranged in a random direction. The ceramic layer 112 has a piezoelectric characteristic, which is polarized in response to an electric signal or an ultrasound signal, thereby being able to transmit and receive the ultrasound signal. The ceramic layer 112 may be formed with a piezoelectric element such as lead zironate titanate (PZT) and the like.

Also, each transducer element 110 further includes a backing layer 111 configured to absorb vibration and the ultrasound signal propagated from the ceramic layer 112 in an opposite direction with respect to a transmission direction of the ultrasound signal when the ceramic layer 112 is excited in response to an electric signal. The backing layer 11 may be formed with a material having acoustic impedance similar to that of the piezoelectric element, which is used to form the ceramic layer 112, to absorb the ultrasound signal propagated thereto.

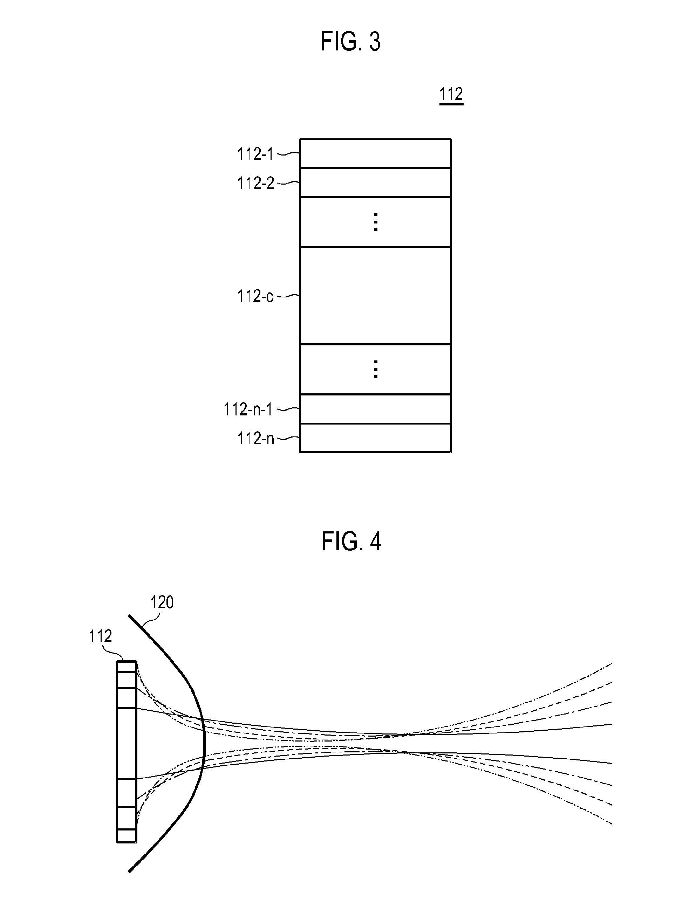

Also, each transducer element further includes a matching layer configured to reduce an acoustic impedance difference between the ceramic layer 112 and a target object. The matching layer includes a first matching layer 113 and a second matching layer 114 covering the ceramic layer 112. The first matching layer and the second matching layer may be formed such that a thickness of each of the first and second matching layer 113 and 114 becomes 1/4 of a wavelength of the ultrasound signal substantially. Further, the acoustic impedance of the first and second matching layers 113 and 114 is an interim value of acoustic impedance of the ceramic layer and the target object. FIG 3 is a schematic diagram showing an illustrative embodiment of the ceramic layer 112 in each transducer element 110. Referring to FIG 2, the ceramic layer 112 is formed by using a plurality of ceramic elements 112_1 to 112_n, which have different thicknesses from each other. The plurality of ceramic elements 112_1 to 112_n may be symmetrically formed in both edge directions with respect to a center ceramic element 112_c. Thicknesses of the ceramic elements may be defined in a longitudinal direction of the transducer element 110.

Generally, when the thickness of a ceramic element is thick, an ultrasound signal generated from the ceramic element has a low frequency characteristic rather than a high frequency characteristic. As such, if the ceramic layer is formed with a single ceramic element, then an ultrasound signal generated therefrom has transmission and reception characteristics of a single frequency range. On the other hand, if the ceramic layer is formed with the plurality of ceramic elements 112_1 to 112_n having different thicknesses, an ultrasound signal generated therefrom has transmission and reception characteristics of a plurality of frequency ranges.

In one embodiment, the plurality of ceramic elements is formed to gradually have a narrower thickness in both edge directions with respect to the center ceramic element 112_c of the ceramic layer 112. FIG 4 is a schematic diagram showing profiles of ultrasound transmit beams when the plurality of ceramic elements is formed to gradually have a narrower thickness in both edge directions with respect to the center ceramic element of the ceramic layer 112.

If the ceramic layer 112 is formed like FIG 4, then an ultrasound signal generated from a center ceramic element 112_c, which is relatively thick, has a low frequency characteristic. This is so that the ultrasound signal may focus on a relatively deeper region. However, ultrasound signals generated from ceramic elements, which are formed with a relatively narrower thickness in both edge directions with respect to the center ceramic element 112_c, have high frequency characteristics so that the ultrasound signals may focus on relatively shallow regions. Thus, the high frequency characteristics, which are deficient in the ultrasound signal generated from the center ceramic element 112_c, may be compensated by using the ultrasound signals having the high frequency characteristics, which are outputted from the ceramic elements at the both edge directions. That is, it may compensate resolution of the ultrasound image for degradation at the shallow regions of the target object due to the low frequency characteristic of the transmit ultrasound beam.

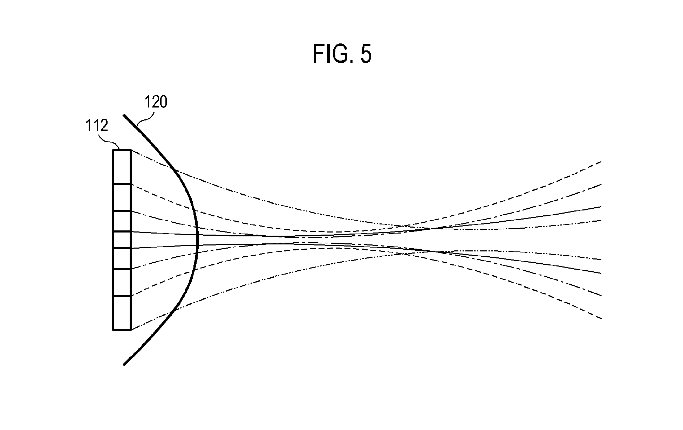

In another embodiment, the plurality of ceramic elements is formed to gradually have a thicker thickness in both edge directions with respect to the center ceramic element 112_c of the ceramic layer 112. FIG 5 is a schematic diagram showing profiles of ultrasound transmit beams when the plurality of ceramic elements is formed to gradually have a thicker thickness in both edge directions with respect to the center ceramic element of the ceramic layer 112.

If the ceramic layer 112 is formed like FIG 5, then an ultrasound signal generated from a center ceramic element 112_c, which is relatively narrow, has a high frequency characteristic. This is so that the ultrasound signal may focus on a relatively shallow region in the target object. However, ultrasound signals generated from ceramic elements, which are formed with a relatively thicker thickness in both edge directions with respect to the center ceramic element 112_c, have low frequency characteristics so that the ultrasound signals may focus on relatively a deep region. Thus, the low frequency characteristic, which is deficient in the ultrasound signal generated from the center ceramic element 112_c, may be compensated by using the ultrasound signals having the low frequency characteristics, which are outputted from the ceramic elements at the both edge directions. That is, it may compensate resolution of the ultrasound image for degradation at the deep region of the target object due to the high frequency characteristic of the transmit ultrasound beam.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound probe for transmitting and receiving ultrasound signals, comprising:
at least one transducer element having a ceramic layer,
wherein the ceramic layer includes a plurality of ceramic elements having different thicknesses.

2. The ultrasound probe of Claim 1, wherein the ceramic layer is formed with a first ceramic element and a plurality of second ceramic elements positioned at both edge sides of the first ceramic element.

3. The ultrasound probe of Claim 2, wherein the plurality of second ceramic elements are formed to gradually have a thicker thickness in both edge directions with respect to the first ceramic element.

4. The ultrasound probe of Claim 2, wherein the plurality of second ceramic elements are formed to gradually have a narrower thickness in both edge directions with respect to the first ceramic element.

5. The ultrasound probe of Claim 2, wherein the ceramic layer is formed with a piezoelectric material.

6. The ultrasound probe of Claim 2, wherein the transducer element further includes:
a backing layer configured to absorb vibration and the ultrasound signal propagated from the ceramic layer in an opposite direction with respect to a transmission direction of the ultrasound signals; and
a matching layer configured to reduce an acoustic impedance difference between the ceramic layer and a target object.

7. The ultrasound probe of Claim 5, wherein the matching layer includes a first matching layer and a second matching layer, wherein a thickness of the respective first matching layer and second matching layer is 1/4 of a wavelength of the ultrasound signal, and wherein acoustic impedance of the first and second matching layers is an interim value of acoustic values of the ceramic layer and the target object.
